**Europäisches Patentamt**

**European Patent Office**

**Office européen des brevets**

(19)

(11) Veröffentlichungsnummer: **0 330 853 B1**

(12) **EUROPÄISCHE PATENTSCHRIFT**

(45) Veröffentlichungstag der Patentschrift: **28.07.93**

(51) Int. Cl.5: **C07C 69/15**, C07C 67/055, B01J 35/02

(21) Anmeldenummer: **89101796.4**

(22) Anmeldetag: **02.02.89**

(54) **Verfahren zur Herstellung von Vinylacetat.**

(30) Priorität: **09.02.88 DE 3803900**

(43) Veröffentlichungstag der Anmeldung:
**06.09.89 Patentblatt 89/36**

(45) Bekanntmachung des Hinweises auf die Patenterteilung:
**28.07.93 Patentblatt 93/30**

(84) Benannte Vertragsstaaten:
**AT BE CH DE ES FR GB GR IT LI NL**

(56) Entgegenhaltungen:
**EP-A- 0 004 079**
**EP-A- 0 012 968**
**EP-A- 0 083 791**
**US-A- 4 668 819**

(73) Patentinhaber: **HOECHST AKTIENGESELL-SCHAFT**
**Postfach 80 03 20**
**W-6230 Frankfurt am Main 80(DE)**

(72) Erfinder: **Wunder, Friedrich, Dr.**
**Jahnstrasse 46**
**W-6093 Flörsheim am Main(DE)**
Erfinder: **Roscher, Günter, Dr.**
**Hölderlinstrasse 64**
**W-6233 Kelkheim (Taunus)(DE)**
Erfinder: **Eichler, Klaus, Dr.**
**Im Traminer 10**
**W-6236 Eschborn 2(DE)**

EP 0 330 853 B1

**Beschreibung**

Es ist bekannt, daß man Ethylen in der Gasphase mit Essigsäure und Sauerstoff oder sauerstoffhaltigen Gasen an Festbettkatalysatoren zu Vinylacetat umsetzen kann. Geeignete Katalysatoren enthalten einen Edelmetallanteil und einen Aktivatoranteil. Der Edelmetallanteil besteht vorzugsweise aus Palladium und/oder dessen Verbindungen; zusätzlich können noch Gold und/oder dessen Verbindungen anwesend sein (US-PS 3 939 199, DE-OS 2 100 778, US-PS 4 668 819). Der Aktivatoranteil besteht dabei aus Verbindungen von Elementen der 1. Hauptgruppe und/oder der 2. Hauptgruppe und/oder Cadmium. Bevorzugt ist Kalium als Element der 1. Hauptgruppe. Diese aktiven Komponenten werden in feiner Verteilung auf Träger aufgebracht, wobei als Trägermaterial im allgemeinen Kieselsäure oder Aluminiumoxid verwendet wird.

Die spezifische Oberfläche der Träger liegt im allgemeinen bei 40-350 $m^2$/g. Nach US-PS 3 939 199 soll das Gesamtporenvolumen bei 0,4-1,2 ml/g liegen, und davon sollen weniger als 10 % von "Mikroporen" mit einem Porendurchmesser unter 30 Angström gebildet werden. Geeignete Träger mit diesen Eigenschaften sind z.B. aerogenes $SiO_2$ oder aerogenes $SiO_2$-$Al_2O_3$-Gemisch. Die Trägerteilchen bei der Vinylacetatherstellung haben im allgemeinen die Form von Kugeln. Aber auch Tabletten und Zylinder wurden bereits eingesetzt.

Gegenüber den bisher technisch am häufigsten verwendeten kugelförmigen Trägern aus gebranntem und mit Säure gewaschenem Bentonit zeigen schon die regulären Zylinder (d.h. solche mit ebenen Stirnflächen) aus aerogenem $SiO_2$ oder $SiO_2$-$Al_2O_3$-Gemisch eine um etwa 20 % erhöhte Raum-Zeit-Ausbeute. Überraschenderweise hat sich nun gezeigt, daß bei Verwendung des genannten aerogenen Ausgangsmaterials zylindrische Teilchen mit gewölbten Stirnflächen gegenüber regulären Zylindern eine nochmals wesentlich erhöhte Raum-Zeit-Ausbeute erbringen.

Gegenstand der Erfindung ist ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, sowie gegebenenfalls zusätzlich Gold und/oder Goldverbindungen, sowie als Aktivatoren Alkaliverbindungen und gegebenenfalls zusätzlich Cadmiumverbindungen auf einem Träger enthält, der aus aerogenem $SiO_2$ oder aerogenem $SiO_2$-$Al_2O_3$-Gemisch mit einer Oberfläche von 50-250 $m^2$/g und einem Porenvolumen von 0,4-1,2 ml/g besteht, wobei das Porenvolumen zu mindestens 50 % von Poren mit einem Durchmesser von 50-200 Angström gebildet wird, dadurch gekennzeichnet, daß der Träger aus zylindrischen Teilchen mit gewölbten Stirnflächen besteht.

Das zur Herstellung der zylindrischen Trägerteilchen verwendete aerogene Oxid oder Oxidgemisch wird beispielsweise durch Flammenhydrolyse von Siliciumtetrachlorid oder einem Siliciumtetrachlorid-Aluminiumtrichlorid-Gemisch in einer Knallgasflamme hergestellt. Die so erhaltenen glasigen "Mikrokugeln" haben eine Oberfläche von 100-300 $m^2$/g. Besonders geeignet sind (unter dem Namen ®Aerosil handelsübliche) Mikrokugeln mit einer Oberfläche von 150 bis 200 $m^2$/g, die aus 97 Gew.-% $SiO_2$ und 3 Gew.-% $Al_2O_3$ bestehen.

Die Mikrokugeln können z.B. auf die Weise zu den erfindungsgemäß eingesetzten zylindrischen Teilchen verarbeitet werden, daß man die Mikrokugeln mit einer verdünnten Lösung mineralischen Leimes anteigt, durch Pressen in die gewünschte zylindrische Form (mit gewölbten Stirnfläche) bringt und den Leim durch Brennen in eine schwerlösliche Form überführt. Als anorganischer Leim kommt beispielsweise Wasserglas, Kieselsäuresol, Aluminiumoxydsol, Kaolin, Bentonit in Frage, wobei Kaolin in Mengen von 1-20 Gew.-%, insbesondere von 3-10 Gew.-% bevorzugt wird.

Nach dem Pressen werden die zylindrischen Teilchen gebrannt, wobei sich ein Porenvolumen von 0,4-1,2 ml/g und eine Oberfläche von 50 bis 250 $m^2$/g ausbildet. Dabei wird das Porenvolumen zumindestens 50 % von Poren mit einem Durchmesser von 50-200 Angström gebildet. Besonders günstig sind Oberfläche von 70 bis 150 $m^2$/g. Die Größe der Oberfäche ist dabei von Brenntemperatur und -dauer abhängig. Je hörer die Temperatur und je länger die Brennzeit ist, umso kleiner werden die Oberflächen.

Durch Verwendung der zylindrischen Trägerteilchen mit gewölbten Stirnfläche gelingt es, die Raum-Zeit-Ausbeute der Katalysatoren bei gleichem Gehalt an aktiven Substanzen und gleichen Reaktionsbedingungen wesentlich zu steigern, ohne daß die Selektivität leidet. Die Vorteile des erfindungsgemäßen Verfahrens liegen darin, daß durch diese Leistungssteigerung bei Neuanlagen Katalysatormenge und Reaktorvolumen verringert werden können, was zu beträchtlichen Verringerungen der Anlagekosten führt, oder daß bei bereits bestehenden Anlagen die Kapazität ohne Umbau wesentlich erhöht werden kann und man so die Investitionskosten für die Anlagenerweiterung einspart. Man kann aber auch stattdessen die Raum-Zeit-Ausbeute unverändert lassen und erhält dann gegenüber den herkömmlichen Katalysatoren bei gleicher Raum-Zeit-Ausbeute eine wesentlich verringerte $CO_2$-Bildung und damit eine höhere Selektivität, wodurch bedeutende Mengen Ethylen eingespart werden.

Bei der Oberfläche der genannten Träger handelt es sich stets um die sogenannte BET-Oberfläche, gemessen nach der Methode von Brunauer, Emmett und Teller. Sie gibt die Gesamtoberfläche von 1g Trägermaterial an, d.h. die Summe aus der äußeren Oberfläche des Trägers und der Oberfläche sämtlicher offenen Poren. Das gesamte Porenvolumen und der Anteil davon, den Poren bestimmter Größe (z.B. diejenigen mit einem Durchmesser von 50-200 Angström) beitragen, läßt sich mit Hilfe der Quecksilber-Porosimetrie messen.

Die Abmessung der zylindrischen Teilchen wird vorzugsweise so gewählt, daß einerseits ein leichtes Einfüllen des Trägers in den Reaktor gewährleistet ist (d.h. Ausschluß extrem großer Teilchen) und andererseits kein großer Druckabfall entsteht (d.h. Ausschluß extrem kleiner Teilchen). Im allgemeinen haben die zylindrischen Teilchen einen Radius von 2-8 mm, vorzugsweise von 2-4 mm. Die Länge der zylindrischen Teilchen (inklusive der gewölbten Stirnfläche) liegt bei 2 bis 15 mm, vorzugsweise bei 6 bis 15 mm.

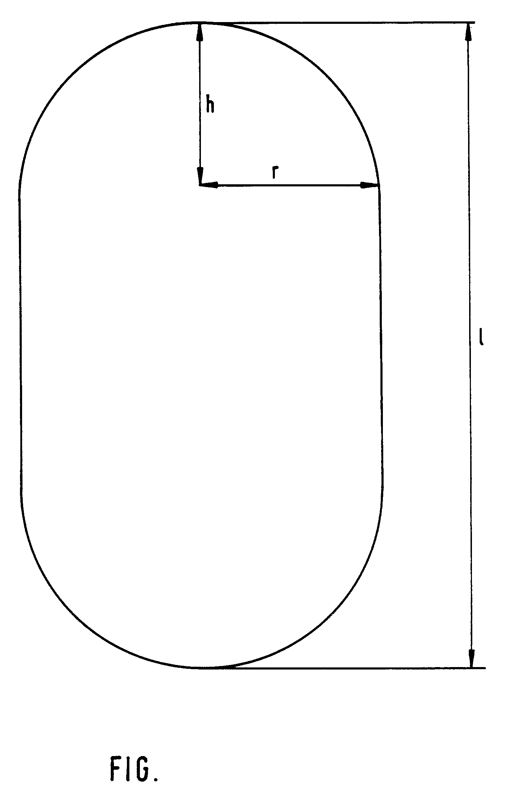

Die Höhe jeder der beiden gewölbten Stirnflächen beträgt das 0,2 bis 1-fache des Zylinderradius. Die Figur zeigt die Form der zylindrischen Teilchen; dabei bedeutet l die Länge (inklusive der gewölbten Stirnfläche), r den Radius des Zylinders und h die Höhe der gewölbten Stirnflächen.

Die Form der zylindrischen Teilchen mit gewölbter Stirnfläche ist vergleichbar der Form der bekannten Arzneimittelkapseln.

Die katalytisch aktiven Substanzen werden in üblicher Weise auf den Träger aufgebracht, beispielsweise durch Tränken des Trägers mit einer Lösung der aktiven Substanzen, anschließende Trocknung und gegebenenfalls Reduktion.

Jedoch kann man die aktiven Substanzen auch beispielsweise durch Ausfällung auf dem Träger, durch Aufsprühen, Aufdampfen, Tauchen aufbringen.

Als Lösungsmittel für die katalytisch aktiven Substanzen sind vor allem unsubstituierte Carbonsäuren mit 2 bis 10 Kohlenstoffatomen im Molekül, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird vorzugsweise Essigsäure als Lösungsmittel eingesetzt. Die zusätzliche Verwendung eines inerten Lösungsmittels ist dann zweckmäßig, wenn die Substanzen in der Carbonsäure nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind. Genannt seien neben Wasser beispielsweise Ketone wie Aceton und Acetylaceton, ferner Ether wie Tetrahydrofuran oder Dioxan, aber auch Kohlenwasserstoffe wie Benzol.

Der Katalysator enthält als Edelmetallkomponente Palladium und/oder dessen Verbindungen und als Aktivatorkomponente Alkaliverbindungen. Als zusätzliche Edelmetallkomponente kann er Gold und/oder dessen Verbindungen enthalten, und als zusätzliche Aktivatorkomponente kann er Cadmiumverbindungen enthalten.

Als Verbindungen des Palladiums kommen alle Salze und Komplexe in Betracht, die löslich (sowie gegebenenfalls reduzierbar) sind und im fertigen Katalysator keine desaktivierenden Stoffe wie Halogen oder Schwefel hinterlassen. Besonders geeignet sind die Carboxylate, vorzugsweise die Salze der aliphatischen Monocarbonsäuren mit 2 bis 5 Kohlenstoffatomen, etwa das Acetat, das Propionat oder das Butyrat. Weiter sind beispielsweise geeignet das Nitrat, Nitrit, Oxidhydrat, Oxalat, Acetylacetonat, Acetoacetat. Aber auch Verbindungen wie das Sulfat und die Halogenide können verwendet werden, wenn man dafür Sorge trägt, daß der Sulfatrest, z.B. durch Fällen mit Bariumacetat, oder das Halogen, z.B. durch Fällen mit Silbernitrat, vor der Tränkung entfernt wird, so daß das Sulfat- oder Halogenanion nicht auf den Träger gerät. Wegen seiner Löslichkeit und seiner Zugänglichkeit ist Palladiumacetat die besonders bevorzugte Palladiumverbindung.

Im allgemeinen liegt der Gehalt an Palladium im Katalysator bei 1,3-3 Gew.-%, vorzugsweise bei 1,5 bis 2,5 Gew.-%, insbesondere bei 2-2,5 Gew.-%, wobei der Metallanteil auf die Gesamtmasse des Trägerkatalysators bezogen wird.

Neben Palladium und/oder dessen Verbindungen können noch zusätzlich Gold und/oder dessen Verbindungen anwesend sein. Eine besonders geeignete Goldverbindung ist Bariumacetoaurat. Im allgemeinen wird Gold bzw. eine seiner Verbindungen, falls es eingesetzt wird, in einem Anteil von 0,2 bis 0,7 Gew.-% zugegeben, wobei der Metallanteil auf die Gesamtmasse des Trägerkatalysators bezogen wird.

Als Aktivatoren enthält der Katalysator Alkaliverbindungen und gegebenenfalls zusätzlich Cadmiumverbindungen. Geeignet sind beispielsweise Alkalicarboxylate wie etwa Kaliumacetat, Natriumacetat, Lithiumacetat, Natriumpropionat. Geeignet sind auch solche Alkaliverbindungen, die unter Reaktionsbedingungen in die Carboxylate übergehen, wie etwa Hydroxide, Oxide, Carbonate. Als Verbindungen des Cadmiums kommen solche in Frage, die kein Halogen oder Schwefel enthalten, beispielsweise Carboxylat (bevorzugt), Oxid, Hydroxid, Carbonat, Citrat, Tartrat, Nitrat, Acetylacetonat, Benzoylacetonat, Acetoacetat. Besonders geeignet ist Cadmiumacetat. Auch Gemische verschiedener Aktivatoren lassen sich einsetzen. Jeder einzelne Aktivator wird im allgemeinen in einem Anteil von 0,5 bis 4 Gew.-% zugegeben. wobei der Metallanteil des Aktivators auf die Gesamtmasse des Trägerkatalysators bezogen wird.

Bevorzugt sind folgende Katalysatoren:

Palladium/Alkalielement/Cadmium sowie Palladium/Gold/Alkalielement, wobei Palladium bzw. Gold als Metalle oder Verbindungen im fertigen Katalysator vorliegen können und wobei als Alkalielement Kalium bevorzugt ist (in Form eines Carboxylats). Das Verhältnis K:Pd bzw. K:(Pd + Au) ist dabei vorzugsweise 0,7:1 bis 2:1. Das Verhältnis Cd:Pd bzw. Cd:(Pd + Au) ist vorzugsweise 0,6:1 bis 2:1, insbesondere 0,6:1 bis 0,9 1. Dabei werden Pd, Au, Cd, K stets als Elemente gerechnet, d.h. z.B. nur die Metallanteile von Pd-Acetat, Cd-Acetat und K-Acetat auf dem Träger miteinander verglichen.

Besonders bevorzugt sind die Katalysatoren Palladiumacetat/Kaliumacetat/Cadmiumacetat, sowie Palladiumacetat/Bariumacetoaurat/Kaliumacetat.

Die Tränkung des Katalysatorträgers mit der Lösung der aktiven Komponenten wird vorzugsweise so vorgenommen, daß das Trägermaterial mit der Lösung überschichtet und die überschüssige Lösung dann abgegossen oder abfiltriert wird. Mit Rücksicht auf Lösungsverluste ist es vorteilhaft, nur die dem integralen Porenvolumen des Katalysatorträgers entsprechende Lösung einzusetzen und sorgfältig durchzumischen, damit alle Teilchen des Trägermaterials gleichmäßig benetzt werden. Diese Durchmischung läßt sich z.B. durch Rühren erreichen. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es zweckmäßig, die Menge und die Zusammensetzung der zum Tränken des Katalysatorträgers verwendeten Lösung so zu bemessen, daß sie dem Porenvolumen des Trägermaterials entspricht und daß durch einmaliges Tränken die gewünschte Menge aktiver Stoffe aufgebracht wird.

Die Trocknung des mit der Lösung der aktiven Stoffe getränkten Katalysatorträgers wird vorzugsweise unter vermindertem Druck durchgeführt. Die Temperatur bei der Trocknung sollte unter $120\,^\circ$C, vorzugsweise unter $90\,^\circ$C liegen. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehalt nach der Trocknung sollte vorzugsweise weniger als 8 Gew.-%, insbesondere weniger als 6 Gew.-% betragen.

Falls eine Reduktion der Palladiumverbindungen (und ggf. der Goldverbindungen) durchgeführt wird, was manchmal nützlich sein kann, so kann diese im Vakuum, bei Normaldruck oder bei erhöhtem Druck bis zu 10 bar ausgeführt werden. Dabei empfiehlt es sich, das Reduktionsmittel umso stärker mit einem Inertgas zu verdünnen, je höher der Druck ist. Die Reduktionstemperatur liegt zwischen 40 und $260\,^\circ$C, vorzugsweise zwischen 70 und $200\,^\circ$C. Im allgemeinen ist es zweckmäßig, für die Reduktion ein Inertgas-Reduktionsmittel-Gemisch zu verwenden, das 0,01 bis 50 Vol-%, vorzugsweise 0,5 bis 20 Vol.-% Reduktionsmittel enthält. Als Inertgas können beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen und andere Olefine in Frage. Die Menge des Reduktionsmittels richtet sich nach der Menge des Palladiums und gegebenenfalls des eingesetzten Goldes; das Reduktionsäquivalent soll mindestens das 1- bis 1,5-fache des Oxydationsäquivalents betragen, jedoch schaden größere Mengen Reduktionsmittel nicht. Beispielsweise soll auf 1 Mol Palladium mindestens 1 Mol Wasserstoff verwendet werden. Die Reduktion kann im Anschluß an die Trocknung in der gleichen Anlage vorgenommen werden.

Die Herstellung des Vinylacetats erfolgt im allgemeinen durch Leiten von Essigsäure, Ethylen und Sauerstoff oder Sauerstoff enthaltenden Gasen bei Temperaturen von 100 bis $220\,^\circ$C, vorzugsweise 120 bis $200\,^\circ$C, und bei Drucken von 1 bis 25 bar, vorzugsweise 1 bis 20 bar, über den fertigen Katalysator, wobei nicht-umgesetzte Komponenten im Kreise geführt werden können. Zweckmäßig hält man die Sauerstoffkonzentration unter 8 Vol.-% (bezogen auf das essigsäurefreie Gasgemisch). Unter Umständen ist jedoch auch eine Verdünnung mit inerten Gasen, wie Stickstoff oder Kohlendioxid vorteilhaft. Besonders $CO_2$ eignet sich zur Verdünnung bei Kreisprozessen, da es in geringen Mengen während der Reaktion gebildet wird.

Die folgenden Beispiele sollen die Erfindung erläutern.

**Vergleichsbeispiel 1** (Kugelförmige Trägerteilchen aus konventionellem, nicht-aerogenem $SiO_2$)

Es wurden 500 g (= 1 l) eines Kieselsäureträgers eingesetzt, der aus gebrannten und dann mit HCl gewaschenem Bentonit ($SiO_2$-Gehalt nach dieser Wäsche 96 Gew.-%) zu Kugeln von 5-6 mm Durchmesser gepreßt worden war. Der Träger aus diesen kugelförmigen Teilchen hatte eine BET-Oberfläche von 128 $m^2$/g und ein Gesamtporenvolumen von 0,77 ml/g, das zu 33 % von Poren mit 50-200 Angström Durchmesser gebildet wurde. Der Träger wurde mit einer (diesem Porenvolumen entsprechenden) Lösung von 20 g Pd-Acetat, 14,5 g Cd-Acetat und 18,5 g K-Acetat in 340 ml Eisessig getränkt und bei 60°C unter Stickstoff bei einem Druck von 270 mbar bis zu einem Lösungsmittel-Restgehalt von 2 Gew.-% getrocknet. Dies ergab eine Dotierung von 1,7 Gew.-% Pd, 1,1 Gew.-% Cd, 1,4 Gew.-% K (Cd:Pd = 0,65:1, K:Pd = 0,82:1).

Es wurden 50 ml des fertigen Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 142°C das umzusetzende Gas über den Katalysator geleitet. Dieses Gas bestand am Reaktoreingang aus 27 Vol.-% Ethylen, 55 Vol.-% $N_2$, 12 Vol.-% Essigsäure und 6 Vol.-% $O_2$. Die Ergebnisse sind aus der Tabelle ersichtlich.

**Vergleichsbeispiel 2** (Zylindrische Trägerteilchen mit ebenen Stirnflächen, hergestellt aus aerogenem $SiO_2$-$Al_2O_3$-Gemisch)

Es wurden 500 g (= 1 l) eines aerogenen Oxidgemischs aus 97 Gew.-% $SiO_2$ und 3 Gew.-% $Al_2O_3$ (im Handel erhältlich unter dem Namen ®Aerosil MOX 170) mit Hilfe von Kaolin zu zylindrischen Trägerteilchen mit ebenen Stirnflächen gepreßt und dann getrocknet. Nach dem anschließenden Brennen hatten die Teilchen einen Radius von 2,5 mm und eine mittlere Länge von 5 mm, ihre BET-Oberfläche war 139 $m^2$/g. Die Teilchen hatten ein Gesamtporenvolumen von 0,75 ml/g, das zu 78 % von Poren mit 50-200 Angström Durchmesser gebildet wurde. Die Trägerteilchen wurden wie in Vergleichsbeispiel 1 getränkt und getrocknet, so daß die gleiche Dotierung vorlag. Anschließend wurde der Katalysator wie in Vergleichsbeispiel 1 getestet. Die Ergebnisse sind aus der Tabelle ersichtlich.

**Beispiel** (Zylindrische Trägerteilchen mit gewölbten Stirnflächen, hergestellt aus aerogenem $SiO_2$-$Al_2O_3$-Gemisch)

Es wurden 500 g des in Vergleichsbeispiel 2 genannten aerogenen Oxidgemischs zu zylindrischen Trägerteilchen gepreßt, die im Unterschied zu Vergleichsbeispiel 2 gewölbte Stirnflächen hatten. Nach dem Brennen hatten die Teilchen einen Radius von 3 mm, eine mittlere Länge von 6,5 mm (inklusive der beiden gewölbten Stirnflächen); die Höhe der beiden gewölbten Stirnflächen war je 1 mm. Die Teilchen hatten eine BET-Oberfläche von 126 $m^2$/g und ein Gesamtporenvolumen von 0,74 ml/g, das zu 77 % von Poren mit einem Durchmesser von 50-200 Angström gebildet wurde. Die Trägerteilchen wurden wie in Vergleichsbeispiel 1 und 2 getränkt und getrocknet, so daß die gleiche Dotierung vorlag. Anschließend wurde der Katalysator wie im Vergleichsbeispiel 1 und 2 getestet. Die Ergebnisse sind aus der Tabelle ersichtlich.

| | Vergleichsbeispiel 1 | Vergleichsbeispiel 2 | Beispiel |
|---|---|---|---|
| Trägermaterial | Nicht-aerogenes SiO$_2$ | Aerogenes SiO$_2$-Al$_2$O$_3$-Gemisch | Aerogenes SiO$_2$-Al$_2$O$_3$-Gemisch |
| Form | Kugeln | Zylinder mit ebenen Stirnflächen | Zylinder mit gewölbten Stirnflächen |
| Selektivität | 92 % | 93 % | 94 % |
| Raum-Zeit-Ausbeute | 400 g/l·h | 542 g/l·h | 640 g/l·h |

**Patentansprüche**

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen,

6

sowie gegebenenfalls zusätzlich Gold und/oder Goldverbindungen, sowie als Aktivatoren Alkaliverbindungen und gegebenenfalls zusätzlich Cadmiumverbindungen auf einem Träger enthält, der aus aerogenem $SiO_2$ oder aerogenem $SiO_2$-$Al_2O_3$-Gemisch mit einer Oberfläche von 50-250 $m^2$/g und einem Porenvolumen von 0,4-1,2 ml/g besteht, wobei das Porenvolumen zu mindestens 50 % von Poren mit einem Durchmesser von 50-200 Angström gebildet wird, dadurch gekennzeichnet, daß der Träger aus zylindrischen Teilchen mit gewölbten Stirnflächen besteht.

2. Verfahren nach Anspruch 1, dadurch gekennzeichnet, daß die zylindrischen Teilchen mit gewölbter Stirnfläche einen Radius von 2-8 mm und eine Länge, inklusive der gewölbten Stirnflächen, von 2-15 mm haben, wobei die Höhe jeder der beiden gewölbten Stirnflächen das 0,2 bis 1-fache des Radius beträgt.

3. Verfahren nach Anspruch 1, dadurch gekennzeichnet, daß die zylindrischen Teilchen mit gewölbter Stirnfläche einen Radius von 2-4 mm und eine Länge, inklusive der gewölbten Stirnflächen, von 6-15 mm haben, wobei die Höhe jeder der beiden gewölbten Stirnflächen das 0,2 bis 1-fache des Radius beträgt.

**Claims**

1. A process for the preparation of vinyl acetate in the gas phase from ethylene, acetic acid and oxygen or oxygen-containing gases on a catalyst which contains palladium and/or compounds thereof and if appropriate additionally gold and/or gold compounds and, as activators, alkali metal compounds and if appropriate additionally cadmium compounds, on a support which consists of aerogenic $SiO_2$ or an aerogenic $SiO_2$-$Al_2O_3$ mixture having a surface area of 50-250 $m^2$/g and a pore volume of 0.4-1.2 ml/g, the pore volume being formed to the extent of at least 50% by pores having a diameter of 50-200 Angstroms, wherein the support consists of cylindrical particles having curved front faces.

2. The process as claimed in claim 1, where in the cylindrical particles with a curved front face have a radius of 2-8 mm and a length, including the curved front faces, of 2-15 mm, the height of each of the two curved front faces being 0.2 times to once the radius.

3. The process as claimed in claim 1, wherein the cylindrical particles with a curved front face have a radius of 2-4 mm and a length, including the curved front faces, of 6-15 mm, the height of each of the two curved front faces being 0.2 times to once the radius.

**Revendications**

1. Procédé pour préparer de l'acétate de vinyle en phase gazeuse à partir d'éthylène, d'acide acétique et d'oxygène ou de gaz oxygénés sur un catalyseur contenant du palladium et/ou ses composés, et éventuellement en outre de l'or et/ou des composés de l'or, et aussi, en tant qu'activateurs, des composés de métaux alcalins et éventuellement aussi des composés du cadmium, le tout sur un support, lequel est constitué de $SiO_2$ aérogène ou d'un mélange de $SiO_2$ aérogène et de $Al_2O_3$ aérogène ayant une aire spécifique de 50 à 250 $m^2$/g pour un volume de pores de 0,4 à 1,2 ml/g, le volume des pores étant formé, pour au moins 50 %, de pores ayant un diamètre de 50 à 200 Å, caractérisé en ce que le support est constitué de particules cylindriques présentant des surfaces en bout incurvées.

2. Procédé selon la revendication 1, caractérisé en ce que les particules cylindriques ayant une surface en bout incurvée ont un rayon de 2 à 8 mm et une longueur, y compris les surfaces en bout incurvées, de 2 à 15 mm, la hauteur de chacune des deux surfaces en bout incurvées étant égale à 0,2 à 1 fois le rayon.

3. Procédé selon la revendication 1, caractérisé en ce que les particules cylindriques ayant une surface en bout incurvée ont un rayon de 2 à 4 mm et une longueur, y compris les surfaces en bout incurvées, de 6 à 15 mm, la hauteur de chacune des deux surfaces en bout incurvées étant égale à 0,2 à 1 fois le rayon.

FIG.